(19) Europäisches Patentamt
European Patent Office
Office européen des brevets

(11) Veröffentlichungsnummer: **0 269 043 B1**

# (12) EUROPÄISCHE PATENTSCHRIFT

(45) Veröffentlichungstag der Patentschrift: **21.08.91**

(51) Int. Cl.5: **C07C 67/333**, C07C 69/533

(21) Anmeldenummer: **87117209.4**

(22) Anmeldetag: **23.11.87**

## (54) Verfahren zur Herstellung von 3-Pentensäureestern aus 2-Pentensäureestern.

(30) Priorität: **27.11.86 DE 3640596**

(43) Veröffentlichungstag der Anmeldung:
**01.06.88 Patentblatt 88/22**

(45) Bekanntmachung des Hinweises auf die Patenterteilung:
**21.08.91 Patentblatt 91/34**

(84) Benannte Vertragsstaaten:
**BE CH DE ES FR GB IT LI NL**

(56) Entgegenhaltungen:

**ARKIV FÖR KEMI Band 13. Nr. 25, 1958, Seiten 239-246, SALO GRONIWITZ: "3-substituted thiophenes preparation and metalation of 3-methoxythiophene"**

**ADVANCED ORGANIC CHEMISTRY McGraw-Hill Kagokusha, Ltd., 2nd edition 1968, pages 678-680; J. March: "Reactions, mechanisms, and structure"**

**PATENT ABSTRACTS OF JAPAN Band 5, Nr. 79 (C56)(751), 23. Mai 1981 & JP-A-56 29543 (MITSUBISHI) 24.03.1981**

**BEILSTEINS HANDBUCH DER ORGANISCHEN CHEMIE Band II, 4. Auflage, 1920, Seiten 426-427, Verlag von Julius Springer, Berlin,**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**
Erfinder: **Hoelderich, Wolfgang, Dr.**
**Mannheimer Strasse 18 c**
**W-6710 Frankenthal(DE)**
Erfinder: **Sauerwald, Manfred, Dr.**
**Gebhardtstrasse 16**
**W-6701 Roedersheim-Gronau(DE)**

**EP 0 269 043 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus Journal of Organic Chemistry Band 33, Seiten 1971ff. (1968) ist bekannt, daß man durch fünfstündige Bestrahlung von 2-Pentensäureestern als 5 %ige Lösung in Pentan 3-Pentensäureester erhält. Ein solches Verfahren eignet sich nicht für die technische Ausführung und zudem müssen große Mengen an Lösungsmittel zurückgewonnen werden. Nach einem anderen in Tetrahedron Letters Band 25, Seiten 5181ff. (1984) beschriebenen Verfahren werden 2-Alkencarbonsäureester bei -78 °C in Tetrahydrofuran in Gegenwart von Kaliumdisilazid zu 3-Alkencarbonsäureestern umgesetzt. Dieses Verfahren ist hinsichtlich der erforderlichen tiefen Temperaturen und des aufwendigen Katalysators für eine technische Ausführung ungeeignet. Ferner ist aus Canadian Journal of Chemistry Band 46, Seiten 2225ff. (1968) ein Verfahren bekannt, bei dem man 2-trans Pentensäuremethylester durch Erhitzen auf 259 °C für einen Zeitraum von 328 Stunden partiell in 3-Pentensäuremethylester umwandelt. Dieses Verfahren hat den Nachteil, daß es außerordentlich zeitaufwendig ist und bei einer technischen Realisierung erheblicher Reaktionsräume bedarf.

Es war deshalb die technische Aufgabe gestellt, ein Verfahren zur Herstellung von 3-Pentensäureestern aus 2-Pentensäureestern zur Verfügung zu stellen, das in kurzer Zeit verläuft und technisch einfach mit leicht zugänglichen Katalysatoren durchführbar ist.

Diese Aufgabe wird gelöst in einem Verfahren zur Herstellung von 3-Pentensäureestern aus 2-Pentensäureestern, bei dem man

a) 2-Pentensäureester der Formel

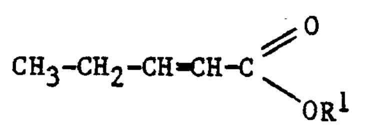

I,

in der $R^1$ einen Alkylrest mit 1 bis 12 Kohlenstoffatomen, einen Cycloalkylrest mit 5 bis 8 Kohlenstoffatomen, einen Aralkylrest mit 7 bis 10 Kohlenstoffatomen oder einen Arylrest mit 6 bis 10 Kohlenstoffatomen bezeichnet, mit Verbindungen der Formel II

$R^2$-X-H   II ,

in der X für ein Sauerstoffatom oder ein Schwefelatom steht und $R^2$ die unter $R^1$ angegebenen Bedeutung hat, oder Verbindungen der Formel III

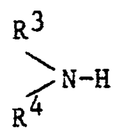

III,

in der $R^3$ ein Wasserstoffatom bezeichnet oder wie $R^4$ für einen Alkylrest mit 1 bis 12 Kohlenstoffatomen, einen Cycloalkylrest mit 5 bis 8 Kohlenstoffatomen, einen Aralkylrest mit 7 bis 10 Kohlenstoffatomen oder einen Arylrest mit 6 bis 10 Kohlenstoffatomen steht, und $R^3$ und $R^4$ gemeinsam mit dem Stickstoffatom, das sie substituieren, einen 5- bis 7-gliedrigen Ring bilden können, der zusätzlich ein weiteres Stickstoff- oder Sauerstoffatom enthalten kann, bei einer Temperatur von 20 bis 300 °C, gegebenenfalls in Gegenwart von basischen Katalysatoren zu Verbindungen der Formel IV

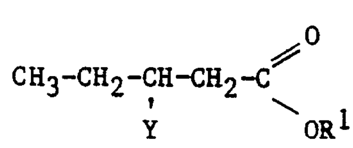

IV,

in der Y die Reste $XR^2$ oder

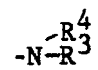

bedeutet, wobei X, $R^1$, $R^2$, $R^3$ und $R^4$ die oben genannte Bedeutung haben, umsetzt und
b) die Verbindungen der Formel IV in der Flüssig- oder Gasphase in Gegenwart von sauren Katalysatoren bei einer Temperatur von 150 bis 450°C zu Gemischen aus 3- und 2-Pentensäureestern spaltet und hieraus 3-Pentensäureester abtrennt.

Das neue Verfahren hat den Vorteil, daß es einfach technisch durchführbar ist. Ferner hat das neue Verfahren den Vorteil, daß es in kurzer Zeit mit guten Ausbeuten verläuft und leicht zugängliche Katalysatoren verwendet werden.

Das neue Verfahren ist insofern bemerkenswert, als bei der Spaltung der in 3-Stellung substituierten Valeriansäureester in Gegenwart von sauren Katalysatoren überwiegend die Bildung von 2-Pentensäureestern zu erwarten war. Ferner war bei der Umsetzung der 2-Pentensäureester mit Aminen die Bildung von Säureamiden zu erwarten.

In den als Ausgangsstoffen verwendeten 2-Pentensäureestern der Formel I bedeutet $R^1$ einen Alkylrest mit 1 bis 12 Kohlenstoffatomen wie Methyl-, Ethyl-, n-Propyl-, iso-Propyl-, n-Butyl, iso-Butyl-, n-Hexyl-, n-Decylrest, Cycloalkylrest mit 5 bis 8 Kohlenstoffatomen, wie Cyclopentyl-, Cyclohexyl-, Cycloheptyl- oder Cyclooctylrest, Aralkylreste mit 7 bis 10 Kohlenstoffatomen wie Benzyl- oder Phenylethylreste, ferner Arylreste mit 6 bis 10 Kohlenstoffatomen, z.B. Phenyl- oder Naphthylreste. Geeignete Ausgangsverbindungen sind beispielsweise 2-Pentensäuremethylester, 2-Pentensäureethylester, 2-Pentensäureisopropylester, 2-Pentensäure-n-butylester, 2-Pentensäurecyclohexylester, 2-Pentensäurebenzylester oder 2-Pentensäurephenylester. Die genannten Pentensäureester können in cis- oder trans-Form vorliegen. Vorteilhaft geht man von 2-Pentensäure-$C_1$-bis-$C_4$-alkylestern aus. Es ist auch möglich, Pentensäureestergemische, die neben 2-Pentensäureestern 3- und 4-Pentensäureester enthalten, zu verwenden. Hierbei werden 2- und 3-Pentensäureester umgesetzt, während 4-Pentensäureester nicht reagiert und leicht abgetrennt werden kann.

Die Umsetzung erfolgt mit Verbindungen der Formel II, in der X für ein Sauerstoffatom oder Schwefelatom steht und $R^2$ die gleiche Bedeutung wie $R^1$ hat. Geeignete Verbindungen sind beispielsweise Methanol, Ethanol, n-Propanol, i-Propanol, n-Butanol, sec.-Butanol, n-Pentanol, Decanol, Cyclohexanol, Cyclopentanol, Cycloheptanol, Phenol, Phenylethanol, Methylmercaptan, Thiophenol. Besondere Bedeutung haben Alkanole mit 1 bis 4 Kohlenstoffatomen erlangt.

Nach einer anderen Arbeitsweise führt man die Umsetzung mit Verbindungen der Formel III durch. In der Formel III bezeichnet $R^3$ ein Wasserstoffatom oder wie $R^4$ einen Alkylrest mit 1 bis 12 Kohlenstoffatomen, einen Cycloalkylrest mit 5 bis 8 Kohlenstoffatomen, einen Aralkylrest mit 7 bis 10 Kohlenstoffatomen oder einen Arylrest mit 6 bis 10 Kohlenstoffatomen. Die Substituenten $R^3$ und $R^4$ können gemeinsam mit dem Stickstoffatom das sie substituieren einen 5- bis 7-gliedrigen Ring bilden, der zudem ein weiteres Stickstoff- oder Sauerstoffatom als Heteroatom enthalten kann. Geeignete Verbindungen sind beispielsweise Methylamin, n-Propylamin, sec.-Propylamin, n-Butylamin, Dimethylamin, Diethylamin, Di-n-butylamin, Piperidin, Morpholin, Piperazin oder Pyrrolidin oder Azazycloheptan. Bevorzugt sind Verbindungen der Formel III, in der $R^3$ ein Wasserstoffatom bezeichnet oder wie $R^4$ für einen Alkylrest mit 1 bis 4 Kohlenstoffatomen, einen Cyclohexylrest oder einen Benzylrest steht, oder $R^3$ und $R^4$ zusammen mit dem Stickstoffatom, den sie substituieren, einen 5- bis 7-gliedrigen Ring bezeichnen, der ein weiteres Sauerstoffatom oder ein Stickstoffatom als Heteroatom enthalten kann.

Bei der Umsetzung von 2-Pentensäureestern der Formel I mit Verbindungen der Formel II ist es zweckmäßig, wenn $R^1$ und $R^2$ den gleichen Rest bezeichnen.

Während für die Umsetzung von 2-Pentensäureestern I mit Verbindungen der Formel III in der Regel keine Katalysatoren verwendet werden, führt man die Umsetzung mit Verbindungen der Formel II zweckmäßig in Gegenwart von basischen Katalysatoren durch.

Geeignete basische Katalysatoren sind z.B. Alkali- oder Erdalkalihydroxide, wie Lithiumhydroxid, Natriumhydroxid, Kaliumhydroxid, Calciumhydroxid, Bariumhydroxid, ferner Alkoholate von Alkali- oder Erdalkalimetallen, insbesondere mit Alkanolen mit 1 bis 4 Kohlenstoffatomen, wie Natriummethylat, Natriumethylat, Magnesiumethylat, ferner Aluminiumalkoholate oder Titanalkoholate sowie stark basische Ionenaustauscher, z.B. vernetztes Polystyrol, das Aminogruppen, insbesondere quarternäre Aminogruppen enthält. Weiterhin sind geeignete Katalysatoren Alkali- oder Erdalkaliamide oder Hydride wie Natriumamid, Kaliumamid, Natriumhydrid, Kaliumhydrid oder Kalziumhydrid. Besonders bevorzugt sind Natrium- oder Kaliumalkoholate von Alkanolen mit 1 bis 4 Kohlenstoffatomen, stark basische Ionenaustauscher, Amide oder Hydride von Natrium, Kalium oder Kalzium.

Das Molverhältnis von 2-Pentensäureester I zu Verbindungen der Formel II oder III beträgt vorteilhaft 1 :

0,5 bis 1 : 10, insbesondere 1 : 1 bis 1 : 5.

Das Molverhältnis von Pentensäureester I zu den basischen Katalysatoren beträgt vorteilhaft 1 : 0,01 bis 1 : 0,5, insbesondere 1 : 0,05 bis 1 : 0,2.

Die Umsetzung führt man bei einer Temperatur von 20 bis 300° C, vorzugsweise 50 bis 200° C durch. Vorteilhaft wählt man bei der Umsetzung von 2-Pentensäureestern I mit Verbindungen der Formel II eine Temperatur von 40 bis 95° C. In der Regel führt man die Umsetzung bei Atmosphärendruck durch, es ist aber auch möglich erhöhten Druck, z.B. bis zu 10 bar, anzuwenden. Die Reaktionszeiten betragen in der Regel 0,1 bis 5, insbesondere 1 bis 2 Stunden.

Die Umsetzung kann diskontinuierlich oder kontinuierlich in flüssiger Phase, z.B. mit homogen gelösten oder in der Flüssigphase suspendieten Katalysatoren, oder auch an fest angeordneten Katalysatoren, z.B. in Sumpf- oder Rieselfahrweise, durchgeführt werden. Andererseits ist es auch möglich, die Umsetzung in der Gasphase durchzuführen.

Die Umsetzung nimmt man bei diskontinuierlicher Arbeitsweise, z.B. wie folgt vor: 2-Pentensäureester I werden mit den oben angegebenen Mengen der Verbindung II unter Mitverwendung der beschriebenen basischen Katalysatoren auf die vorgenannte Temperatur für den genannten Zeitraum erhitzt. Werden 2-Pentensäureester I mit Verbindungen der Formel III umgesetzt, so fällt die Mitverwendung von basischen Katalysatoren weg.

Gegebenenfalls nach Abtrennung der mitverwendeten Katalysatoren, z.B. durch Filtration, Neutralisation und Extraktion mit Wasser wird das Reaktionsgemisch durch Destillation getrennt. Hierbei werden überschüssige Verbindungen II oder III sowie nicht umgesetzte Pentenester-Isomere abgetrennt. Als Reaktionsprodukt erhält man Verbindungen der Formel IV

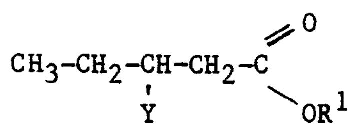

IV

in der Y für die Reste -X-$R^2$ oder

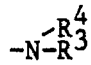

steht, wobei X und $R^1$ bis $R^4$ die obengenannte Bedeutung haben.

Beispielhaft seien genannt: 3-Methoxyvaleriansäuremethylester, 3-Ethoxyvaleriansäureethylester, 3-Propoxyvaleriansäurebutylester, 3-Cyclohexyloxyvaleriansäurecyclohexylester, 3-Benzyloxyvaleriansäurebenzylester, N-Methyl-3-aminovaleriansäuremethylester, N-Butyl-3-aminovaleriansäurebutylester, N-Dimethyl-3-aminovaleriansäuremethylester, N-Dibutyl-3-aminovaleriansäuremethylester, N-Cyclohexyl-3-aminovaleriansäureethylester, N-Benzyl-3-aminovaleriansäuremethylester, 3-Piperidinovaleriansäuremethylester, 3-Morpholinovaleriansäuremethylester oder 3-Piperazinovaleriansäuremethylester.

In der Stufe b) werden die Verbindungen der Formel IV in der Flüssig-oder Gasphase in Gegenwart von sauren Katalysatoren bei einer Temperatur von 150 bis 450° C zu Gemischen aus 3- und 2-Pentensäureestern gespalten und hieraus die gewünschten 3-Pentensäureester abgetrennt, z.B. durch Destillation. Die als Spaltprodukte anfallenden Verbindungen der Formel II und III sowie 2-Pentensäureester und nicht umgesetzte Verbindungen der Formel IV können wiederverwendet werden.

Geeignete saure Katalysatoren sind z.B. sauer wirkende Oxide von Elementen der 3. bis 5. Hauptgruppe sowie der 4. bis 6. Nebengruppe des Periodischen Systems. Besonders bevorzugt sind Siliciumdioxid in Form von Kieselgel, Kieselgur oder Quarz, ferner Titandioxid, Zirkondioxid, Phosphorpentoxid, Vanadiumpentoxid, Bortrioxid, Aluminiumoxid, Chromoxide, Molybdänoxide, Wolframoxide oder Gemische derselben.

Besonders geeignet sind weiterhin zeolithische Katalysatoren. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall, z.B. durch Alkali- oder Wasserstoffionen ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere drei- und zweiwertige Elemente wie B,

Ga, Fe, Cr, Be, As, Sb in das Gitter eingebaut werden oder das Silicium kann durch ein vierwertiges Element wie Ge, Ti, Zr, Hf, ersetzt werden.

Als Katalysatoren kommen Zeolithe aus der Mordenit-Gruppe oder Faujasit-Gruppe wie Y-, X- und L-Zeolith oder engporige Zeolithe vom Erionit-bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzung aufweisen. Es handelt sich hierbei um Alumino-, Boro-, Eisen-, Gallium-, Chrom-, Beryllium-, Arsen-, Antimon- und Wismutsilikatzeolithe oder deren Gemische sowie um Alumino-, Boro-, Gallium- und Eisengermanatzeolithe oder deren Gemische.

Insbesondere eignen sich die Alumino-, Boro- und Eisensilikatzeolithe des Pentasil-Typs für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin-oder 1,3-Propandiamin- oder Triethylentetramin-Lösung mit oder insbesondere ohne Alkali- oder Erdalkalizusatz bei 100 bis 220° C unter autogenem Druck hergestellt. Auch gehören hierzu die isotaktischen Zeolithe nach DE-OS 30 06 471. Die erhaltenen Aluminiumsilikatzeolithe enthalten je nach Wahl der Einsatzstoffmenge ein $SiO_2/Al_2O_3$-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200° C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz zur Reaktion bringt. Auch gehören hierzu die isotaktischen Zeolithe nach DE-OS 30 06 471. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol, durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200° C unter autogenem Druck.

Die so hergestellten Alumino-, Boro- und Eisensilikatzeolithe können nach ihrer Isolierung, Trocknung bei 100 bis 160° C, vorzugsweise 110 bis 150° C und Calcinierung bei 450 bis 550° C, vorzugsweise 500 bis 540° C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 95 : 5, bevorzugt 75 : 25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110° C/16 h getrocknet und bei 500° C/16 h calciniert.

Man erhält auch vorteilhaft Katalysatoren, wenn der isolierte Alumino-bzw. Borosilikat direkt nach der Trocknung verformt wird und erstmals nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolitischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$Gemisch bei 400 bis 550° C, bevorzugt 500 bis 540° C, zu regenerieren. Die Zeolithe erhalten dadurch ihre Anfangsaktivität zurück. Durch partielle Verkokung (pre-coke) ist es möglich die Aktivität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe mit 0,1 bis 1 Gew.% Metallen z.B. der VIII. Nebengruppe oder I. Gruppe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert.

Zweckmäßigerweise führt man die Dotierung so durch, daß man z.B. den verformten Pentasil-Zeolithen in einem Steigrohr vorlegt und bei 20 bis 100° C z.B. eine wäßrige oder ammoniakalische Lösung eines Halogenids oder eines Nitrats der Metalle überleitet. Ein derartiger Ionenaustausch kann z.B. an der

Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermahlige Calcinierung an.

Eine mögliche Ausführungsform besteht z.B. darin, daß man $Cs_2CO_3$ in Wasser löst. Mit dieser Lösung wird der verformte oder unverformte Zeolithe eine gewisse Zeit, ca. 30 Minuten, getränkt. Die eventuell überstehende Lösung wird am Rotationsverdampfer von Wasser befreit. Danach wird der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei ca. 150 °C getrocknet und bei ca. 550 °C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Weitere Katalysatoren für die Herstellung von 3-Pentensäureestern aus Verbindungen der Formel IV sind Phosphate der Elemente Al, B, Zr, Ce, Fe oder deren Gemische.

Als Aluminiumphosphat-Katalysatoren werden für das erfindungsgemäße Verfahren gefällte und insbesondere unter hydrothermalen Bedingungen synthetisierte Aluminiumphosphate eingesetzt.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit in Wasser homogen mischt; zu dieser Mischung Tetrapropylammoniumhydroxid gibt und danach bei ca. 150 °C 20 bis 60 h unter autogenem Druck in einem Autoklaven umsetzt. Das abfiltrierte $AlPO_4$-5 wird getrocknet bei 100 bis 160 °C und calciniert bei 450 bis 550 °C.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP 103 117, US 4 440 871 beschrieben. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

Die hier beschriebenen Katalysatoren können wahlweise als 2- bis 4-mm-Stränge oder als Tabletten mit 3 bis 5 mm Durchmesser oder als Pulver mit Teilchengrößen von 0,1 bis 0,5 mm oder als Wirbelkontakt eingesetzt werden.

Die für das erfindungsgemäße Verfahren in der Regel gewählten Reaktionsbedingungen sind in der bevorzugten Gasphase 150 bis 450 °C, vorzugsweise 180 bis 300 °C, und eine Belastung WHSV = 0,05 bis 20 $h^{-1}$, bevorzugt 0,3 bis 5 $h^{-1}$ (g Edukt/g Katalysator x h).

Schwerflüchtige Edukte werden in gelöster Form z.B. in THF-, Toluol-, Methanol- oder Petrolether-Lösung zum Einsatz gebracht. Generell ist auch eine Verdünnung mit Lösungsmitteln wie z.B. voranstehend genannt oder Inertgasen wie $N_2$, Ar, möglich.

Eine Ausführungsform des erfindungsgemäßen Verfahrens in der Gasphase sieht z.B. so aus, daß man die jeweilige Verbindung IV zunächst verdampft und dann, gegebenenfalls zusammen mit einem Inertgas wie Stickstoff, Kohlendioxid oder Argon, bei der vorgenannten Reaktionstemperatur gasförmig über einen fest angeordneten oder aber einen in auf- und abwirbelnder Bewegung befindlichen Katalysator leitet. Der Reaktionsaustrag wird mittels geeigneter Kühlvorrichtung kondensiert und anschließend durch fraktionierende Destillation aufgearbeitet. Hierbei trennt man den gewünschten 3-Pentensäureester von 2-Pentensäureester I und gegebenenfalls von nicht umgesetzter Verbindung IV ab. Die 2-Pentenester können in die Stufe a) zurückgeführt werden.

Nach einer anderen Arbeitsweise wird die Reaktion gemäß Stufe b) in flüssiger Phase, z.B. in einem hochsiedenden Öl mit einem Siedebereich oberhalb der Siedepunkte des Einsatzstoffes IV bzw. der Eliminierungsprodukte durchgeführt.

Die Abspaltungsreaktion wird z.B. durch Einleiten der Verbindungen IV unter die Oberfläche eines Öls bei Temperaturen über dem Siedepunkt der Eliminierungsprodukte durchgeführt. Die Eliminierungsprodukte sowie gegebenenfalls nicht umgesetztes Ausgangsmaterial werden gasförmig abgezogen, während ein Teil des mit schwerflüchtigen Nebenprodukten angereicherten Öls ausgeschleust und durch frisches Öl ersetzt wird, wobei man das ausgeschleuste Öl nicht wiedergewinnt, sondern der Unterfeuerung zwecks Energiegewinnung zuführt. Als Reaktionsprodukte werden hauptsächlich 3-Pentensäureester (cis und trans) und als Nebenprodukte 2-trans-Pentensäureester erhalten, neben wenig 4-Pentensäureester und 2-cis-Pentensäureester.

Als Reaktionsmedium werden in der Regel unter Reaktionsbedingungen inerte, hochsiedende Kohlenwasserstoffe, insbesondere entsprechende Mineralölfraktionen mit Siedebereichen zwischen 300 und 550 °C verwendet. In Betracht kommen beispielsweise Vakuumgasöl, schweres Heizöl, Vakuumrückstand, technisches Weißöl, Marlothermöl oder geschmolzenes Paraffinwachs.

Die Reaktionstemperaturen liegen im allgemeinen bei 100 bis 450 °C, vorzugsweise 150 bis 350 °C, insbesondere bei 200 bis 300 °C, wobei man in der Regel Normaldruck oder Unterdruck anwendet. Es ist jedoch auch möglich, bei Überdruck zu arbeiten.

Die Abspaltungsreaktion wird in Anwesenheit von Katalysatoren durchgeführt. Als Katalysatoren sind sowohl im Reaktionsmedium lösliche als auch unlösliche saure Verbindungen geeignet, die demgemäß entweder gelöst, emulgiert oder suspendiert vorliegen. Bevorzugt verwendet man aliphatische oder aromatische Sulfonsäuren wie Benzolsulfonsäure, Toluolsulfonsäure oder Dodecylbenzolsulfonsäure, anorganische Säuren wie Schwefelsäure, Borsäure, Phosphorsäure oder deren partiell veresterte Derivate, ferner Diphenylphosphinsäure und Säureanhydride wie Phosphorpentoxid oder Boroxid. Ebenfalls geeignet sind saure Katalysatoren auf Trägern, z.B. Phosphorsäure auf Kieselgel sowie Aluminiumoxid, Aluminiumphosphat, Borphosphat, Aluminiumsilikat, Herteropolysäuren des Phosphors, Molybdän- und Wolframsäure.

Die Katalysatoren werden dem hochsiedenden Öl in Mengen von 0,01 bis 25 Gew.%, vorzugsweise 0,1 bis 10 Gew.%, insbesondere 1 bis 5 Gew.% zugesetzt.

Die Umsetzung wird vorzugsweise kontinuierlich durchgeführt. Als Reaktoren sind beispielsweise Rührbehälter oder zylindrische Reaktoren oder Füllkörperkolonnen geeignet. Diese sind zweckmäßig bis zu 2/3 mit dem als Reaktionsmedium verwendeten Öl, dem gegebenenfalls Katalysatoren zugesetzt werden, beschickt. Das Ausgangsprodukt wird vorteilhaft von unten, eventuell mit einem Inertgas wie Stickstoff als Strippgas dem Reaktor zugeführt. Das Reaktionsgemisch wird auf der vorgenannten Reaktionstemperatur gehalten und die ausgetragenen Produkte durch Abkühlung kondensiert. Die Aufarbeitung der Reaktionsprodukte kann nach bekannten Methoden, z.B. durch Destillation oder Extraktion, erfolgen. Geringe Mengen schwerflüchtiger Nebenprodukte können durch partielles Abziehen des Reaktionsmediums ausgeschleust werden, wobei der Reaktorinhalt, falls erforderlich, mit frischem Öl ergänzt wird. Eine Aufarbeitung und Rückführung des abgezogenen Öls ist in der Regel nicht wirtschaftlich, da die verwendeten Öle wohlfeil zur Verfügung stehen. Man wird daher zweckmäßig das ausgeschleuste Öl der Energiegewinnung durch Unterfeuerung zuführen.

Dieses neue Verfahren hat im Vergleich zu anderen bekannten Flüssigphasenreaktionen den Vorteil, daß unmittelbar mit der Umsetzung eine Stofftrennung verbunden ist, d.h. die Wertprodukte werden in dem Maße, wie sie entstehen, aus dem Reaktionsmedium entfernt, während schwerflüchtige Nebenprodukte (Crackprodukte, Polymere usw.) im Öl zurückbleiben. Aufgrund der kurzen Verweilzeiten und geringen Produktkonzentration werden Nebenreaktionen weitgehend unterdrückt. Ferner ist das Verfahren technisch einfach durchführbar.

3-Pentensäureester stellen wichtige Zwischenprodukte, z.B. für die Herstellung von Adipinsäure und Caprolactam dar.

Beispiel 1

In einem 500-ml-Dreihalskolben wurden 228 g 2-trans-Pentensäuremethylester bei Raumtemperatur mit einer Lösung von 5,4 g Natriummethylat in 320 g Methanol (Molverhältnis 1 : 0,05 : 5) vermischt, auf 65 °C erwärmt und 4 Stunden bei dieser Temperatur gerührt. Nach dem Abkühlen wurde das Natriummethylat mit Eisessig neutralisiert, das Methanol bei Normaldruck weitgehend abdestilliert und der Rückstand anschließend fraktionierend destilliert. Hierbei wurden 211,9 g 3-Methoxivaleriansäuremethylester (73 % d. Th.) von Siedepunkt 121 bis 126 °C/302 mbar erhalten. Außerdem fielen 40,1 g unumgesetzter 2-Pentensäuremethylester und 4,6 g Destillationsrückstand an.

Beispiel 2

Wurde nicht 2-trans-, sondern 2-cis-Pentensäuremethylester eingesetzt, so wurden ausgehend von 57,7 g 2-cis-Pentensäuremethylester, 1,35 g Natriummethylat und 80 g Methanol unter den Reaktions- und Aufarbeitungsbedingungen von Beispiel 1 44,2 g 3-Methoxivaleriansäuremethylester (61 % d. Th.) von Siedepunkt 123 bis 128° C/300 mbar erhalten. Außerdem wurden 17,6 g 2-Pentensäuremethylester gewonnen. Der Destillationsrückstand betrug 1,5 g.

Beispiel 3

Wurde Beispiel 1 mit 5,6 g KOH anstelle von 5,4 g Natriummethylat wiederholt, so wurden unter den Reaktions- und Aufarbeitungsbedingungen von Beispiel 1 100,9 g 3-Methoxivaleriansäuremethylester von Siedepunkt 122 bis 125° C/304 mbar (35 % d. Th.) und 131,1 g Pentensäuremethylester (58 % d. Th.) erhalten.

Beispiel 4

In einem Gemisch aus 100 g Pentensäuremethylester (85 % 2-trans-, 5 % 2-cis- und 10 % 3-Pentensäuremethylester) und 140 g Methanol wurden 56 g eines starkbasischen Ionenaustauschers (OH-Form, methanolfeucht) suspendiert. Nach Erwärmen auf 65° C wurde 3 Stunden bei dieser Temperatur gerührt. Nach dieser Zeit zeigte die gaschromatographische Analyse, daß das Reaktionsgemisch (ohne Methanol) zu 80,3 % aus 3-Methoxivaleriansäuremethylester und zu 18,8 % aus Pentenestern (12 % 2-trans-, 0,5 % 2-cis- und 6,3 % 3-Pentensäuremethylester) bestand (Flächen-%). Durch fraktionierende Destillation wurden 93,4 g 3-Methoxivaleriansäuremethylester (73 % d. Th.) von Siedepunkt 128° C/302 mbar und 15,3 g Pentensäuremethylester (15 % d. Th.) erhalten.

Beispiel 5

Eine Lösung von 0,81 g Natriummethylat in 17,1 g 2-trans-Pentensäuremethylesterund 16,5 g Thiophenol wurde 2 Stunden auf 65° C erwärmt. Nach dieser Zeit zeigte die gaschromatographische Analyse 82 % 3-Thiophenoxivaleriansäuremethylester, 8 % Thiophenol und 4 % 2-trans-Pentensäuremethylester. Durch Destillation wurden 15,2 g 3-Thiophenoxivaleriansäuremethylester mit einem Siedepunkt von ca. 100° C/1 mbar (45 % d. Th.) erhalten.

Beispiel 6

Ein Gemisch aus 500 g 2-trans-Pentensäuremethylester und 372 g Piperidin wurde 24 Stunden lang auf 100° C erhitzt. Durch fraktionierende Destillation wurden 562 g 3-Piperidino-valeriansäuremethylester vom Siedepunkt 75 bis 77° C/2 mbar (64 % d. Th.) erhalten.

Beispiel 7

Ein Gemisch aus 9 g 4- und 1 g 2-cis-Pentensäuremethylester wurde mit 0,024 g Natriummethylat in 1,4 g Methanol versetzt und 3 Stunden lang auf 100° C erhitzt. Laut gaschromatographischer Analyse bestand das Reaktionsgemisch nach dieser Zeit zu 89,3 % aus 4-Pentensäuremethylester, zu 0,2 % aus 2-cis-, zu 3,3 % aus 2-trans-, zu 2 % aus 3-Pentensäuremethylester und zu 5,1 % aus 3-Methoxivaleriansäuremethylester. Dies Beispiel zeigt, daß 4-Pentensäuremethylester unverändert bleibt, während 2-Pentensäureester umgesetzt wird.

Beispiel 8

Pro Stunde wurden 10 g 3-Methoxivaleriansäuremethylester in einen Verdampfer gepumpt und von dort gasförmig zusammen mit 3 l Stickstoff bei 300° C über 5 g eines $Al_2O_3$-Katalysators geleitet. Die gasförmigen Reaktionsausträge wurden kondensiert, gewogen und gaschromatographisch analysiert. Innerhalb von 6 Stunden Reaktionszeit wurden 49 g Reaktionsaustrag erhalten, der zu 37 % aus 3-, zu 33 % aus 2-trans-, zu 5 % aus 2-cis-, zu 1 % aus 4-Pentensäuremethylester und zu 22 % aus unumgesetztem 3-Methoxivaleriansäuremethylester bestand. Das Verhältnis von 3- zu 2-Pentenestern betrug demnach 1 : 1.

Beispiel 9

Pro Stunde wurden 10 g 3-Methoxivaleriansäuremethylester in einen Verdampfer gepumpt und von dort gasförmig zusammen mit 3 l Stickstoff bei 350 °C über 5 g eines $SiO_2$-Katalysators geleitet. Die gasförmigen Reaktionsausträge wurden kondensiert, gewogen und gaschromatographisch analysiert. Innerhalb von 6 Stunden wurden 53,3 g Reaktionsaustrag erhalten, der zu 30 % aus 3-, zu 56 % aus 2-trans-, zu 8 % aus 2-cis-, zu 2 % aus 4-Pentensäuremethylester und zu 3 % aus unumgesetztem 3-Methoxivaleriansäuremethylester bestand. Das Verhältnis von 3- zu 2-Pentenestern betrug demnach 1 : 2,2.

Beispiel 10

Wie in Beispiel 9 beschrieben, wurden 10 g Piperidinovaleriansäuremethylester aus a) bei 300*C über 5 g Aluminiumoxid geleitet. Der Reaktionsaustrag bestand zu 10 % aus 3-, zu 24 % aus 2-Pentensäuremethylester, zu 8 % aus Ausgangsprodukt und zu 16 % aus unbekannten Verbindungen.

In den Beispielen 11 bis 15 ist die Herstellung von Zeolithen beschrieben, die für die Spaltung von 3-Methoxivaleriansäuremethylester verwendet wurden.

Beispiel 11

Katalysator A

Der Aluminosilikatzeolith vom Pentasil-Typ wurde unter hydrothermalen Bedingungen bei autogenem Druck und 150 °C aus 650 g hochdispersem $SiO_2$, 203 g $Al_2(SO_4)_3$ x 18 $H_2O$ in 10 kg einer wäßrigen 1,6-Hexadiamin-Lösung (Mischung 50 : 50 Gew.%) in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Aluminosilikatzeolith enthielt 92,8 Gew.% $SiO_2$ und 4,2 Gew.% $Al_2O_3$.

Katalysator A wird erhalten, indem man den reinen Aluminosilikatzeolithen des Pentasil-Typs mit Verformungshilfsmitteln zu 2-mm-Strängen verformt, bei 110 °C/16 h getrocknet und bei 500 °C/24 h calciniert.

Beispiel 12

Katalysator B

Der Borosilikatzeolith des Pentasil-Typs wird in einer hydrothermalen Synthese aus 640 g hochdispersem $SiO_2$, 122 g $H_3BO_3$, 8 kg einer wäßrigen 1,6-Hexandiamin-Lösung (Mischung 50 : 50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit Verformungshilfsmitteln 2-mm-Stränge hergestellt, die bei 110 °C/16 h getrocknet und bei 500 °C/24 h calciniert werden.

Beispiel 13

Katalysator C

Katalysator C wird erhalten, indem man Katalysator B mit wäßriger $Cs_2CO_3$-Lösung imprägniert. Nach Trocknung bei 130 °C/2 h und Calcination bei 540 °C/2 h beträgt der Cs-Gehalt 0,6 Gew.%.

Beispiel 14

Katalysator D

Katalysator D wird erhalten, indem man den Aluminosilikatzeolithen von Katalysator A mit Boehmit im Gewichtsverhältnis 60 : 40 verformt, bei 110 °C trocknet und bei 500 °C/16 h calciniert. Die hierbei erhaltenen Stränge werden mit wäßriger LiOH-Lösung imprägniert. Nach einer Trocknung bei 130 °C/2 h und Calcination bei 540 °C/2 h beträgt der Li-Gehalt 0,9 Gew.%.

Beispiel 15

Katalysator E

Kommerziell erhältlicher Na-Y-Zeolith wird mit wäßriger $(NH_4)_2SO_4$-Lösung nach bekannter Methode ionenausgetauscht, so lange bis der Na-Gehalt kleiner 0,5 Gew.% liegt (nach Trocknung 110 °C/2 H und Calcination bei 570 °C/3 h).

Das so erhaltene Pulver wird mit Verformungshilfsmitteln zu Strängen verformt, bei 110 °C getrocknet und bei 500 °C/16 h calciniert.

Beispiele 16 bis 22

Die Beispiele 16 bis 22 wurden unter isothermen Bedingungen in einen Rohrreaktor (Durchmesser 0,6 cm, Länge 90 cm) in der Gasphase mit Zeolithen als Katalysatoren durchgeführt. Eingesetzt wurde nach a) hergestellter 3-Methoxivaleriansäuremethylester. Als Lösungsmittel wurden THF oder Methanol verwendet. Die Reaktionsprodukte wurden gaschromatographisch analysiert (Tabelle).

Beispiel 23

Ein 1-l-Rührkolben wurde mit 500 g Vakuumgasöl sowie 6 g einer 85 %igen Phosphorsäure beschickt und aus 300 °C beheizt. Aus einem Vorratsgefäß wurden stündlich 40 g 3-Methoxivaleriansäuremethylester zusammen mit 10 l Stickstoff unter der Oberfläche des gerührten Öl/Katalysator-Gemisches zugeführt. Die das Reaktionsgefäß verlassenden gasförmigen Produkte wurden abgekühlt und das Kondensat gaschromatographisch untersucht. Stündlich erhielt man so neben 7,5 nicht umgesetztem 3-Methoxivaleriansäuremethylester u.a. 11,0 g 3-Pentensäuremethylester und 10,7 g 2-trans-Pentensäureester.

Tabelle:
Rückspaltung von 3-Methoxivaleriansäuremethylester (3-MVSE) an Zeolithen

| Beispiel | 16 | 17 | 18 | 19 | 20 | 21 | 22 |
|---|---|---|---|---|---|---|---|
| Edukt[1)] | I | I | I | II | II | II | II |
| Katalysator | B | D | E | A | C | E | E |
| Temperatur | 300°C | 300°C | 180°C | 180°C | 300°C | 180°C | 300°C |
| WHSV | 2 $h^{-1}$ | 2 $h^{-1}$ | 2,5 $h^{-1}$ | 2,6 $h^{-1}$ | 3,6 $h^{-1}$ | 2,8 $h^{-1}$ | 2,3 $h^{-1}$ |
| Reaktionsaustrag enthält in Gew.%: | | | | | | | |
| 3-PSE[2)] | 42,9 | 46,2 | 44 | 40,6 | 38,7 | 45,3 | 44,7 |
| 2-trans-PSE | 34,4 | 28,0 | 10,5 | 24,5 | 36,5 | 12,2 | 28,0 |
| 2-cis-PSE | 7,1 | 6,5 | 1,6 | 4,7 | 12,0 | 1,7 | 8,7 |
| 4-PSE | 8,6 | 9,9 | 5,5 | 7,9 | 5,9 | 5,7 | 8,3 |
| 5-Methyl-butyrolacton | 5,6 | 3,9 | 19,3 | 7,3 | 1,0 | 17,8 | 4,4 |
| 3-MVSE | / | / | 18,4 | 13,9 | 5,8 | 16,2 | 5,2 |

1) I: 3-MVSE zu THF = 1 : 1 (Gew.%); II: 3-MSVE zu $CH_3OH$ = 1 : 1 (Gew.%)
2) Pentensäuremethylester = PSE

## Patentansprüche

1. Verfahren zur Herstellung von 3-Pentensäureestern aus 2-Pentensäureestern, dadurch gekennzeichnet, daß man

a) 2-Pentensäureester der Formel

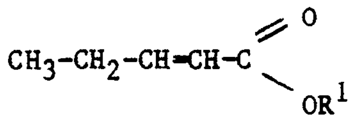 I,

in der $R^1$ einen Alkylrest mit 1 bis 12 Kohlenstoffatomen, einen Cycloalkylrest mit 5 bis 8 Kohlenstoffatomen, einen Aralkylrest mit 7 bis 10 Kohlenstoffatomen oder einen Arylrest mit 6 bis 10 Kohlenstoffatomen bezeichnet, mit Verbindungen der Formel II

$R^2$-X-H II,

in der $R^2$ die für $R^1$ angegebene Bedeutung hat und X für ein Sauerstoff- oder Schwefelatom steht oder mit Verbindungen der Formel III

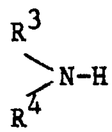 III,

in der $R^3$ ein Wasserstoffatom bezeichnet oder wie $R^4$ für einen Alkylrest mit 1 bis 12 Kohlenstoffatomen, einen Cycloalkylrest mit 5 bis 8 Kohlenstoffatomen, einen Aralkylrest mit 7 bis 10 Kohlenstoffatomen oder einen Arylrest mit 6 bis 10 Kohlenstoffatomen steht, und $R^3$ und $R^4$ gemeinsam mit dem Stickstoffatom, das sie substituieren, einen 5- bis 7-gliedrigen Ring bilden können, der zusätzlich ein Stickstoff- oder Sauerstoffatom als Heteroatom enthalten kann, bei einer Temperatur von 20 bis 300 °C, gegebenenfalls in Gegenwart von basischen Katalysatoren, zu Verbindungen der Formel IV

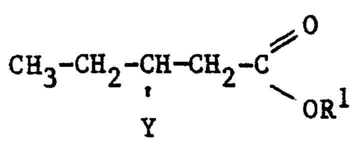 IV,

in der Y für den Rest -X$R^2$ oder

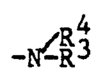

steht, wobei X sowie $R^1$ bis $R^4$ die oben genannte Bedeutung haben, umsetzt und

b) die Verbindungen der Formel IV in der Flüssig- oder Gasphase in Gegenwart von sauren Katalysatoren bei einer Temperatur von 150 bis 450 °C zu Gemischen aus 3- und 2-Pentensäureestern spaltet und 3-Pentensäureester abtrennt.

**2.** Verfahren nach Anspruch 1, dadurch gekennzeichnet, daß man bei der Umsetzung von 2-Pentensäureestern der Formel I mit Verbindungen der Formel II als basische Katalysatoren Alkali- oder Erdalkalihydroxide, Alkalialkoholate oder stark basische Ionenaustauscher mitverwendet und die Umsetzung bei einer Temperatur von 40 bis 95 °C durchführt.

**3.** Verfahren nach den Ansprüchen 1 und 2, dadurch gekennzeichnet, daß man je Mol 2-Pentensäureester der Formel I 1 bis 5 Mol Verbindungen der Formel II oder III verwendet.

4. Verfahren nach den Ansprüchen 1 bis 3, dadurch gekennzeichnet, daß man in der Stufe b) als saure Katalysatoren Aluminiumoxid, Bortrioxid, Titandioxid, Siliciumdioxid, Zeolithe oder Phosphate oder Gemische derselben verwendet.

5. Verfahren nach den Ansprüchen 1 bis 4, dadurch gekennzeichnet, daß man in der Stufe b) als saure Katalysatoren Zeolithe des Faujasit-Typs verwendet.

6. Verfahren nach den Ansprüchen 1 bis 4, dadurch gekennzeichnet, daß man in der Stufe b) als saure Katalysatoren Aluminiumsilikatzeolithe des Pentasil-Typs verwendet.

7. Verfahren nach den Ansprüchen 1 bis 4, dadurch gekennzeichnet, daß man in der Stufe b) als saure Katalysatoren Borosilikatzeolithe des Pentasil-Typs verwendet.

8. Verfahren nach den Ansprüchen 1 bis 4, dadurch gekennzeichnet, daß man als saure Katalysatoren in der Stufe b) Eisensilikatzeolithe des Pentasil-Typs verwendet.

9. Verfahren nach den Ansprüchen 1 bis 4, daduroh gekennzeichnet, daß man in der Stufe b) als saure Katalysatoren Aluminiumphosphate, Siliciumaluminiumphosphate, Eisensiliciumphosphate oder Borphosphate oder deren Gemische verwendet.

10. Verfahren nach den Ansprüchen 1 bis 4, dadurch gekennzeichnet, daß man in der Stufe b) als saure Katalysatoren Zeolithe, die mit Alkali-, Erdalkali- oder Übergangsmetallen oder seltenen Erdmetallen oder deren Gemischen dotiert sind, verwendet.

11. Verfahren nach den Ansprüchen 1 bis 4, dadurch gekennzeichnet, daß man in der Stufe b) Verbindungen der Formel IV in einem hochsiedenden Öl bei einer Temperatur oberhalb des Siedepunkts der jeweils angewandten Verbindung der Formel IV und deren Spaltprodukte durchführt, die Reaktionsprodukte gasförmig abzieht, einen Teil des schwerflüchtige Nebenprodukte enthaltenden Öls ausschleust und durch frisches Öl ersetzt.

12. Verfahren nach Anspruch 11, dadurch gekennzeichnet, daß man als hochsiedendes Öl Mineralöl mit dem Siedepunkt von 300 bis 550 °C verwendet.

13. Verfahren nach den Ansprüchen 11 und 12, dadurch gekennzeichnet, daß man als hochsiedendes Öl Vakuumgasöl, schweres Heizöl, Vakuumrückstand, technisches Weißöl oder geschmolzenes Paraffinwachs verwendet.

14. Verfahren nach den Ansprüchen 11 bis 13, dadurch gekennzeichnet, daß man die Umsetzung bei Atmosphärendruck und/oder unter vermindertem Druck durchführt.

## Claims

1. A process for the preparation of a 3-pentenoate from a 2-pentenoate, wherein
   a) a 2-pentenoate of the formula

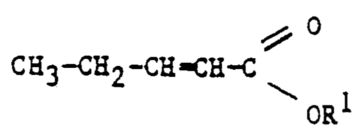

I

where $R^1$ is alkyl of 1 to 12 carbon atoms, cycloalkyl of 5 to 8 carbon atoms, aralkyl of 7 to 10 carbon atoms or aryl of 6 to 10 carbon atoms, is reacted with a compound of the formula II

$R^2$-X-H II

where $R^2$ has the meanings given for $R^1$ and X is an oxygen or sulfur atom, or with a compound of

the formula III,

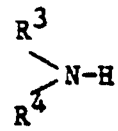

III

where $R^3$ is hydrogen or $R^3$ and $R^4$ are each alkyl of 1 to 12 carbon atoms, cycloalkyl of 5 to 8 carbon atoms, aralkyl of 7 to 10 carbon atoms or aryl of 6 to 10 carbon atoms, and $R^3$ and $R^4$ together with the nitrogen atom on which they are substituents may form a 5-membered to 7-membered ring which may additionally contain a nitrogen or oxygen atom as a hetero atom, at from 20 to 300 °C, in the presence or absence of a basic catalyst, to give a compound of the formula IV

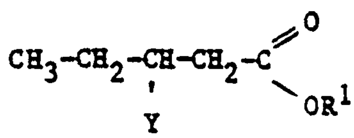

IV

where Y is $-XR^2$ or

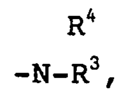

in which X, $R^1$, $R^2$ $R^3$ and $R^4$ have the above meanings, and
b) the compound of the formula IV is cleaved in the liquid or gas phase in the presence of an acidic catalyst at from 150 to 450 °C to give a mixture of 3- and 2-pentenoates, and the 3-pentenoate is isolated.

**2.** A process as claimed in claim 1, wherein, in the reaction of a 2-pentenoate of the formula I with a compound of the formula II, an alkali metal or alkaline earth metal hydroxide, an alkali metal alcoholate or a strongly basic ion exchanger is used as the catalyst and the reaction is carried out at from 40 to 95 °C.

**3.** A process as claimed in claim 1 or 2, wherein from 1 to 5 moles of a compound of the formula II or III is used per mole of 2-pentenoate of the formula I.

**4.** A process as claimed in claim 1 or 2 or 3, wherein, in stage b), alumina, boron trioxide, titanium dioxide, silica, a zeolite or a phosphate or a mixture of these is used as the catalyst.

**5.** A process as claimed in claim 1 or 2 or 3 or 4, wherein, in stage b), a zeolite of the faujasite type is used as the catalyst.

**6.** A process as claimed in claim 1 or 2 or 3 or 4, wherein, in stage b), an aluminosilicate zeolite of the pentasil type is used as the catalyst.

**7.** A process as claimed in claim 1 or 2 or 3 or 4, wherein, in stage b), a borosilicate zeolite of the pentasil type is used as the catalyst.

**8.** A process as claimed in claim 1 or 2 or 3 or 4, wherein, in stage b), an iron silicate zeolite of the pentasil type is used as the catalyst.

**9.** A process as claimed in claim 1 or 2 or 3 or 4, wherein, in stage b), an aluminum phosphate, a silicon aluminum phosphate, an iron silicon phosphate or a boron phosphate or a mixture of these is used as

the acidic catalyst.

**10.** A process as claimed in claim 1 or 2 or 3 or 4, wherein, in stage b), a zeolite which is doped with alkali, alkaline earth or transition metals or rare earth metals or a mixture of these is used as the acidic catalyst.

**11.** A process as claimed in claim 1 or 2 or 3 or 4, wherein, in stage b), a compound of the formula IV is cleaved in a high-boiling oil at above the boiling point of the particular compound of the formula IV employed and its cleavage products, the reaction products are removed in gaseous form, and some of the oil containing sparingly volatile byproducts is separated off and replaced with fresh oil.

**12.** A process as claimed in claim 11, wherein a mineral oil having a boiling point of from 300 to 550 °C is used as the high-boiling oil.

**13.** A process as claimed in claims 11 and 12, wherein vacuum gas oil, heavy fuel oil, vacuum residue, industrial white oil or molten paraffin wax is used as the high-boiling oil.

**14.** A process as claimed in claim 11 or 12 or 13, wherein the reaction is carried out under atmospheric pressure and/or under reduced pressure.

## Revendications

**1.** Procédé de preparation d'esters d'acide 3-penténoïque à partir d'esters d'acide 2-penténoïque, caractérisé en ce que

a) on fait réagir, à une temperature de 20 à 300 °C et éventuellement en présence de catalyseurs basiques, des esters d'acide 2-penténoïque de formule

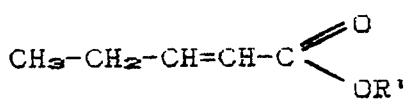

I

dans laquelle $R^1$ désigne un reste alkyle à 1-12 atones de carbone, un reste cycloalkyle à 5-8 atomes de carbone, un reste aralkyle à 7-10 atomes de carbone ou un reste aryle à 6-10 atomes de carbone, avec des composes de formule II

$R^2$-X-H    II

dans laquelle $R^2$ a la signification donnée pour $R^1$ et X est mis pour un atome d'oxygène ou de soufre, ou avec des composés de formule III

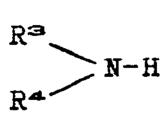

III

dans laquelle $R^3$ designe un atome d'hydrogène ou, de même que $R^4$, est mis pour un reste alkyle à 1-12 atomes de carbone, un reste cycloalkyle à 5-8 atones de carbone, un reste aralkyle à 7-10 atomes de carbone ou un reste aryle à 6-10 atomes de carbone, et $R^3$ et $R^4$ peuvent former, en combinaison avec l'atome d'azote qu ils substituent, un noyau penta- à heptagonal qui peut contenir en plus, en tant qu'hétéroatome, un atome d'azote ou d'oxygène, pour obtenir des composés de formule IV

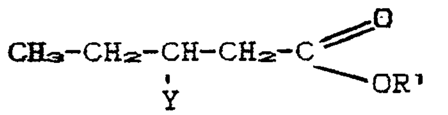

IV

dans laquelle Y est mis pour le reste $-XR^2$ ou

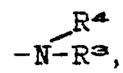

X et $R^1$ à $R^4$ ayant les significations données précédemment, et

b) on scinde les composés de formule IV, en phase liquide ou gazeuse, en présence de catalyseurs acides et à une température de 150 à 450 °C, en mélanges d'esters d'acides 3- et 2-penténoïques et on sépare l'ester d'acide 3-penténoïque.

**2.** Procédé selon la revendication 1, caractérisé en ce que, dans la réaction d'esters d'acide 2-penténoïque de formule I avec des composés de formule II, on utilise simultanément, en tant que catalyseurs, des hydroxydes alcalins ou alcalino-terreux, des alcoolates alcalins ou des échangeurs d'ions fortement basiques, et on conduit la réaction à une température de 40 à 95 °C.

**3.** Procédé selon la revendication 1 ou 2, caractérisé en ce qu'on utilise, par mole d'ester d'acide 2-penténoïque de formule I, de 1 à 5 moles de composés de formule II ou III.

**4.** Procédé selon l'une quelconque des revendications 1 à 3, caractérisé en ce qu'on utilise, comme catalyseur dans l'étape b), de l'oxyde d'aluminium, du trioxyde de bore, du dioxyde de titane, du dioxyde de silicium, des zéolithes ou des phosphates ou des mélanges de ces composés.

**5.** Procédé selon l'une quelconque des revendications 1 à 4, caractérisé en ce qu'on utilise, comme catalyseur dans l'étape b), des zéolithes du type faujasite.

**6.** Procédé selon l'une quelconque des revendications 1 à 4, caractérisé en ce qu'on utilise, comme catalyseur dans l'étape b), des zéolithes d'aluminosilicate du type pentasil.

**7.** Procédé selon l'une quelconque des revendications 1 à 4, caractérisé en ce qu'on utilise, comme catalyseur dans l'étape b), des zéolithes de borosilicate du type pentasil.

**8.** Procédé selon l'une quelconque des revendications 1 à 4, caractérisé en ce qu'on utilise, code catalyseur dans l'étape b), des zéolithes de ferrosilicate du type pentasil.

**9.** Procédé selon l'une quelconque des revendications 1 à 4, caractérisé en ce qu'on utilise, comme catalyseur acide dans l'étape b), des phosphates d'aluminium, des phosphates de silicium et d'aluminium, des phosphates de fer et de silicium, des phosphates de bore ou des mélanges de ces phosphates.

**10.** Procédé selon l'une quelconque des revendications 1 à 4, caractérisé en ce qu'on utilise, comme catalyseur acide dans l'étape b), des zéolithes qui sont dopées avec des métaux alcalins, alcalinoterreux ou de transition, avec des métaux des terres rares ou avec des mélanges de ces métaux.

**11.** Procédé selon l'une quelconque des revendications 1 à 4, caractérisé en ce que, dans l'étape b), on envoie des composés de formule IV dans une huile de point d'ébullition élevé, à une température supérieure au point d'ébullition du composé de formule IV utilisé en particulier et de ses produits de dissociation, on extrait les produits de la réaction à l'état gazeux, on chasse une partie de l'huile contenant des produits secondaires de faible volatilité et on la remplace par de l'huile fraîche.

**12.** Procédé selon la revendication 11, caractérisé en ce qu'on utilise, comme huile de point d'ébullition élevé, une huile minérale dont le point d'ébullition se situe entre 300 et 550 °C.

**13.** Procédé selon la revendication 11 ou 12, caractérisé en ce qu'on utilise, comme huile de point d'ébullition élevé, une huile vacuum, un fuel lourd, un résidu court de la distillation poussée, une huile blanche technique ou une cire de paraffine fondue

**14.** Procédé selon l'une quelconque des revendications 11 à 13, caractérisé en ce qu'on conduit la réaction à la pression atmosphérique et/ou sous pression réduite.